# EUROPEAN PATENT SPECIFICATION

(11) **EP 4 029 898 B1**
(45) Date of publication and mention of the grant of the patent: **06.08.2025**
(21) Application number: 20864031.8
(22) Date of filing: 31.08.2020
(51) Int. Cl.: C08G 65/26, C07C 69/34

(54) **FLUOROPOLYETHER GROUP CONTAINING COMPOUND AND METHOD FOR PRODUCING SAME**
FLUORPOLYETHERGRUPPE ENTHALTENDE VERBINDUNG UND VERFAHREN ZU IHRER HERSTELLUNG
COMPOSÉ CONTENANT UN GROUPE FLUOROPOLYÉTHER ET SON PROCÉDÉ DE PRODUCTION

(30) Priority: 13.09.2019 JP 2019167534; 22.01.2020 JP 2020008580
(43) Date of publication of application: 20.07.2022
(73) Proprietor: Daikin Industries, Ltd., Osaka-shi, Osaka 530-0001 (JP)
(72) Inventor: HIRAGA, Kentarou, Osaka-shi, Osaka 530-8323 (JP); SUGIYAMA, Akinari, Osaka-shi, Osaka 530-8323 (JP); YAMAGUCHI, Fumihiko, Osaka-shi, Osaka 530-8323 (JP); NOMURA, Takashi, Osaka-shi, Osaka 530-8323 (JP); SHIBUTANI, Shouta, Osaka-shi, Osaka 530-8323 (JP); OKUNO, Shingo, Osaka-shi, Osaka 530-8323 (JP)
(74) Representative: Hoffmann Eitle
(86) International application number: PCT/JP2020/032914
(87) International publication number: WO 2021/049361

(56) References cited:
- EP-A2- 0 148 482
- JP-A- 2002 037 880
- JP-A- 2014 517 131
- JP-A- S53 132 519
- JP-A- S60 137 928
- SOLOSKI, E. J. ET AL.: "SYNTHESIS OF PERFLUORO(POLYETHER) DIFUNCTIONAL COMPOUNDS", JOURNAL OF FLUORINE CHEMISTRY, vol. 11, 1978, pages 601 - 612, XP000983897, DOI: 10.1016/S0022-1139(00)81619-X

## Description

### Technical Field

The present disclosure relates to a fluoropolyether group-containing compound and a method for producing the same.

### Background Art

Fluoropolyether group-containing compounds are widely used as e.g. surface-treating agents and lubricants, and their applications have been further expanded.

Ring-opening polymerization of a cyclic ether such as tetrafluorooxetane or hexafluoropropylene oxide, for example, is known as one of the methods for producing a fluoropolyether group-containing compound. However, with such a method, a compound having a functional group only at one end of the molecular chain is usually obtained, and it is difficult to obtain a compound having a functional group at both ends of the molecular chain.

EP-A-0 148 482 relates to a process for preparing halogen-containing polyether, comprising ring-opening polymerizing 2,2,3,3-tetrafluorooxetane in an aprotic solvent in the presence of an alkali metal halide or an alkali metal fluoride and an acyl fluoride, which reacts with the alkali metal fluoride to generate alkoxy anion, or trimethylamine as polymerization initiator, and optionally fluorinating and/or chlorinating the resulting polyether product.

E.J.Soloski et al., J. Fluorine Chem., 11, 601-6012 (1978) describes describes the conversion of Ω-iodoperfluoro (polyether) esters of the formula IR_{f}OR_{f}CO₂R via zinc coupling reactions to yield alpha, omega-perfluoro(polyether) diesters as starting materials for the synthesis of a variety of other difunctional compounds of high molecular weight and exhibiting a variation of oxygen-carbon ratio.

### Summary of Invention

### Technical Problem

Conventionally, in order to obtain a fluoropolyether group-containing compound having a functional group at both ends of the molecular chain, it has been necessary to use a special initiator (JP-A-2002-37880). In particular, it has been difficult to obtain a fluoropolyether group-containing compound having different functional groups at both ends by such a production method.

An object of the present disclosure is to provide a novel fluoropolyether group-containing compound and a method for producing the same.

### Solution to Problem

The present invention provides a compound, which is a fluoropolyether-containing compound of formula (1a) or (1b):

R^{a}-R²-R¹-R^{b} (1a)

R^{a}-R²-R¹-R^{b} (1b)

wherein
- R^{a}, R^{b}: each independently are COF, COOR¹¹, CH₂OH or CHO, provided that R^{a} and R^{b} are not the same; and R¹¹ each independently is H or C₁₋₆-alkyl optionally substituted with F;
- R¹: is C₂₋₁₀-alkylene optionally substituted with F; and
- R²: is -R^{6a}-(OR^{5a})ₙ-O-, wherein R^{5a} is linear C₂₋₁₀-fluoroalkylene, R^{6a} is linear C₁₋₉-fluoroalkylene, and n is an integer of 2-200,
provided that in formula (1a) R^{a} and R^{b} are not the same, and in formula (1b) R^{5a} is CF₂CF₂CH₂ and R^{6a} is CF₂CH₂.

Also, the present invention provides a method for producing a compound of formula (A):

FOC-R²R¹-COOR¹² (A)

wherein
- R¹: is C₂₋₁₀-alkylene optionally substituted with F;
- R²: is -R⁶-(OR⁵)ₙ-O-, wherein R⁵ is linear C₂₋₁₀-fluoroalkylene, R⁶ is linear C₁₋₉-fluoroalkylene, and n is an integer of 2-200; and
- R¹²: is C₁₋₆-alkylene optionally substituted with F;
comprising reacting an acid fluoride compound of the formula (a) :

FOC-R¹³-COOR¹² (a)

wherein
- R¹³: is C₁₋₉-alkylene optionally substituted with F; and
- R¹²: is C₁₋₆-alkylene optionally substituted with F,
with a cyclic ether compound of formula (b): 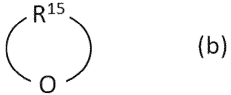 wherein R¹⁵ is linear C₂₋₁₀-fluoroalkylene.

Preferred embodiments of the invention are as defined in the appended dependent claims and/or in the following detailed description.

### Advantageous Effect of Invention

The present invention provides a novel fluoropolyether group-containing compound (also referred to as "the present compound" hereinafter) that can be used in various applications. Further, by using a specific initiator, a fluoropolyether group-containing compound having functional groups at both ends can be easily produced.

### Description of Embodiments

Hereinafter, the present compound will be described in detail.

The present invention provides a fluoropolyether group-containing compound of the following formula (1a) or (1b):

R^{a}-R²-R¹-R^{b} (1a)

R^{a}-R²-R¹-R^{b} (1b)

wherein
- R^{a}, R^{b}: each independently are COF, COOR¹¹, CH₂OH or CHO, wherein R¹¹ each independently is H or C₁₋₆-alkyl optionally substituted with F;
- R¹: is C₂₋₁₀-alkylene optionally substituted with F; and
- R²: is -R^{6a}-(OR^{5a})ₙ-O-, wherein R^{5a} is linear C₂₋₁₀-fluoroalkylene, R^{6a} is linear C₁₋₉-fluoroalkylene, and n is an integer of 2-200,
provided that (i) R^{a} and R^{b} are not the same, and/or (ii) R^{5a} is CF₂CF₂CH₂ and R^{6a} is CF₂CH₂.

In one embodiment, in the present compound of formula (1a) R^{a} and R^{b} are not the same.

In one embodiment, in the present compound of formula (1b) R^{5a} is CF₂CF₂CH₂ and R^{6a} is CF₂CH₂.

The formulas (1a) and (1b) are different in that R^{a} and R^{b} are different in the formula (1a), whereas R^{a} and R^{b} may be the same or different in the formula (1b) and R^{5a} is CF₂CF₂CH₂ and R^{6a} is CF₂CH₂ in formula (1b).

In the formula, R^{a} is COF, COOR¹¹, CH₂OH, or CHO, preferably COF or COOR¹¹, and more preferably COF.

In the formula, R^{a} is COF, COOR¹¹, CH₂OH, or CHO, and preferably COOR¹¹.

In the formula, R¹¹ each independently is H or C₁₋₆-alkyl optionally substituted with F, and preferably C₁₋₆-alkyl optionally substituted with F. The C₁₋₆-alkyl in R¹¹ is preferably C₁₋₃-alkyl, more preferably methyl or ethyl, and still more preferably methyl.

In formula (1a), R^{a} and R^{b} are groups different from each other.

In formula (1b), R^{a} and R^{b} may be the same group or groups different from each other. In one embodiment, R^{a} and R^{b} are the same, and in another embodiment they are different.

In one embodiment, the combination of R^{a} and R^{b} is COF and COOR¹¹ or COOR¹¹ and COOR¹¹, and preferably COF and COOR¹¹.

In the formulae (1a) and (1b), R¹ is C₂₋₁₀-alkylene optionally substituted with F. The C₂₋₁₀-alkylene in R¹ is preferably C₂₋₆-alkylene, more preferably C₂₋₃-alkylene, and still more preferably C₂-alkylene.

In one embodiment, the alkylene group in R¹ is fluoroalkylene, and preferably perfluoroalkylene.

In a preferred embodiment, R¹ is linear C₁₋₃-perfluoroalkylene, and more preferably -CF₂CF₂-.

In formula (1a), R² is -R^{6a}-(OR^{5a})ₙ-O-.

R^{5a} is linear C₂₋₁₀-fluoroalkylene, preferably C₂₋₆-fluoroalkylene, more preferably C₂₋₄-fluoroalkylene, and still more preferably C₃-fluoroalkylene.

In a preferred embodiment, R^{5a} is CF₂CF₂CH₂ or CF₂CF₂CF₂ preferably CF₂CF₂CH₂.

In the formula, R^{6a} is a linear C₁₋₉-fluoroalkylene, preferably C₁₋₅-fluoroalkylene, more preferably C₁₋₃-fluoroalkylene, for example, C₁₋₂-fluoroalkylene.

In a preferred embodiment, R^{6a} is CF₂CH₂ or CF₂CF₂, preferably CF₂CH₂.

In one embodiment, the number of carbon atoms in R^{5a} is one greater than the number of carbon atoms in R^{6a}. For example, when the number of carbon atoms in R^{5a} is 3, the number of carbon atoms in R^{6a} is 2.

In the formula (1b), R^{5a} is CF₂CF₂CH₂ and R^{6a} is CF₂CH₂

In the formulae (1a) and (1b), n is an integer of 2-200, preferably 5-100, and more preferably 10-60.

In a preferred embodiment, in the present compound of formula (1a),
- R^{a}: is COF;
- R^{b}: is COOR¹¹;
- R¹¹: is C₁₋₆-alkylene optionally substituted with F;
- R¹: is C₂₋₆-fluoroalkylene;
- R^{5a}: is linear C₂₋₄-fluoroalkylene;
- R^{6a}: is linear C₁₋₃-fluoroalkylene; and
- n: is an integer of 5-100.

In a more preferred embodiment, in the present compound of formula (1a),
- R^{a}: is COF;
- R^{b}: is COOR¹¹;
- R¹¹: is C₁₋₆-alkylene optionally substituted with F;
- R¹: is C₂₋₆-fluoroalkylene;
- R²: is -CF₂CH₂-(OCF₂CF₂CH₂)ₙ-O-; and
- n: is an integer of 5-100.

In a preferred embodiment, in the present compound of formula (1b),
- R^{a}: is COF or COOR¹¹;
- R^{b}: is COOR¹¹;
- R¹¹: is C₁₋₆-alkyl;
- R¹: is C₂₋₆-fluoroalkylene; and
- n: is an integer of 5-100.

The number-average molecular weight of the fluoropolyether group-containing compound is not limited, but may be, for example, ≥ 500, preferably ≥ 1,000, for example ≥ 3,000, ≥ 5,000, or ≥ 10,000. The number-average molecular weight of the fluoropolyether group-containing compound is not limited, but may be, for example, ≤ 100,000, ≤ 50,000, ≤ 30,000, ≤ 10,000, or ≤ 5,000.

The dispersion (weight-average molecular weight / number-average molecular weight) of the fluoropolyether group-containing compound is not limited, but may preferably be ≤ 1.5, more preferably ≤ 1.3, still more preferably ≤ 1.2, and particularly preferably ≤ 1.1.

The number-average molecular weight and the weight-average molecular weight can be determined by F-NMR, and may also be determined by GPC.

The present disclosure provides a composition containing two or more compounds of the formulae (1a) and/or (1b).

In one embodiment, two or more compounds in the composition have different structures in R^{a} and/or R^{b}. For example, R^{b} may be -COO-alkyl in one compound and R^{b} may be - COOH in the other compound.

The present compound can be suitably used as e.g. a surface-treating agent and a lubricating oil, and an intermediate thereof.

The present invention provides a method for producing the fluoropolyether group-containing compound (also referred to as "the present method" hereinafter).

That is, the present invention provides a method for producing a compound of formula (A):

FOC-R²-R¹-COOR¹² (A)

wherein
- R¹: is C₂₋₁₀-alkylene optionally substituted with F;
- R²: is -R⁶-(OR⁵)ₙ-O-, wherein R⁵ is linear C₂₋₁₀-fluoroalkylene, R⁶ is linear C₁₋₉-fluoroalkylene, and n is an integer of 2-200; and
- R¹²: is C₁₋₆-alkylene optionally substituted with F;
comprising reacting an acid fluoride compound of the formula (a) :

FOC-R¹³-COOR¹² (a)

wherein
- R¹³: is C₁₋₉-alkylene optionally substituted with F; and
- R¹²: is C₁₋₆-alkylene optionally substituted with F,
with a cyclic ether compound of formula (b): 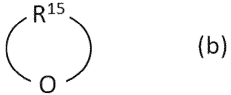 wherein R¹⁵ is linear C₂₋₁₀-fluoroalkylene.

According to the present method, the cyclic ether of the formula (b) is bonded to the COF side of the acid fluoride compound of the formula (a). That is, in the present method, the reaction proceeds at one end side of the acid fluoride compound of the formula (a), and the polymer chain extends at such an end. Therefore, it is possible to obtain a compound having a different functional group, that is, one of COF and COOR¹², at each end of the polymer chain. Further, the present method facilitates the adjustment of the degree of polymerization, molecular weight and degree of dispersion, for example, to obtain a compound with the desired molecular weight or to obtain a compound with a small degree of dispersion, because the reaction is easily controlled.

The formula (A) corresponds to the formula (1a) or (1b) in which R^{a} is COF and R^{b} is COOR¹¹ (where R¹¹ is C₁₋₆-alkyl), and among the symbols of the formula (A), R¹ and R² have the same meaning as the description of formulae (1a) or (1b).

In formula (A), R¹² corresponds to R¹¹ of the formula (1a) and (1b) and is C₁₋₆-alkyl optionally substituted with F. The C₁₋₆-alkyl in R¹² is preferably C₁₋₃-alkyl, more preferably methyl or ethyl, and still more preferably methyl. In one embodiment, the alkyl is substituted with fluorine and is preferably perfluoroalkyl. In another embodiment, the alkyl group is not substituted.

In formula (a), R¹² is C₁₋₆-alkyl optionally substituted with F, and has the same meaning as described above.

In formula (a), R¹³ is C₁₋₉-alkylene optionally substituted with F. R¹ of formula (A) corresponds to a group in which a carbon atom derived from COF of the formula (a) is bonded to the left end of R¹³.

The C₁₋₉-alkylene in R¹³ is preferably C₁₋₅-alkylene, more preferably C₁₋₂-alkylene, and still more preferably methylene.

In one embodiment, the alkylene in R¹³ is fluoroalkylene, and preferably perfluoroalkylene.

In a preferred embodiment, R¹³ is linear C₁₋₂-perfluoroalkylene, and more preferably -CF₂-.

The acid fluoride compound of formula (a) functions as a polymerization initiator in the reaction.

The acid fluoride compound of formula (a) is commercially available or can be produced by a method known to those skilled in the art.

In formula (b), R¹⁵ is linear C₂₋₁₀-fluoroalkylene, preferably an C₂₋₆-fluoroalkylene, more preferably an C₂₋₄-fluoroalkylene, and still more preferably C₃-fluoroalkylene.

In a preferred embodiment, R¹⁵ is CF₂CF₂CH₂ or CF₂CF₂CF₂, preferably CF₂CF₂CH₂.

In a preferred embodiment, the cyclic ether compound of formula (b) is tetrafluorooxetane.

The cyclic ether compound of formula (b) is cleaved between C and O to react with the acid fluoride compound of the above formula (a), resulting in the fluoropolyether group-containing compound of formula (A). That is, the compound of formula (b) forms a portion of R² of formula (A), which is a product.

The compound of formula (b) functions as a monomer in the reaction.

The compound of formula (b) is commercially available or can be produced by a method known to those skilled in the art.

The proportion of the compound of formula (a) to the compound of formula (b) can be appropriately set according to e.g. the molecular weight of the target compound, and for example, the molar ratio may be (0.01:100)-(10:100), preferably (0.1:100)-(5:100), and for example, (1:100)-(5:100).

The reaction between the compound of formula (a) and the compound of formula (b) is usually carried out in a solvent. Examples of the solvent include cyclic ethers such as tetrahydrofuran (THF), tetrahydropyran, and dioxane, cyclic ethers such as diethyl ether, diisopropyl ether, dibutyl ether, monoglyme, diglyme, triglyme, and tetraglyme, aromatic compounds such as HMPA (hexamethylphosphamide), dimethylpropylene (DMPU), tetramethylethylenediamine (TMEDA), toluene, xylene, and benzotrifluorides, amides such as N,N-dimethylformamide, N,N-dimethylacetamide, and N-methylpyrrolidone, and fluorine-containing organic solvents such as hexafluoropropylene, trichlorotrifluoroethane, 1,1,1,3,3-pentafluorobutane, m-xylene hexafluorolide, perfluorohexane, perfluorooctane, perfluorodimethylcyclohexane, perfluorodecalin, perfluoroalkyl ethanol, perfluorobenzene, perfluorotoluene, perfluoroalkylamine (such as fluorinert (trade name)), perfluoroalkyl ether, perfluorobutyl tetrahydrofuran, polyfluoroaliphatic hydrocarbon (AsahiKlin AC6000 (trade name)), hydrochlorofluorocarbon (such as AsahiKlin AK-225 (trade name)), hydrofluoroether (such as Novec (trade name), HFE-7100 (trade name)), 1,1,2,2,3,3,4-heptafluorocyclopentane, fluorinated alcohol, perfluoroalkyl bromide, perfluoroalkyl iodide, perfluoropolyester (such as Krytox (trade name), Demnum (trade name), Fomblin (trade name)), 1,3-bistrifluoromethylbenzene, 2-(perfluoroalkyl)ethyl methacrylate, 2-(perfluoroalkyl)ethyl acrylate, perfluoroalkyl ethylene, Freon 134a, and hexafluoropropene oligomers, or mixtures thereof.

The above reaction is preferably carried out in the presence of a catalyst. Examples of the catalyst include inorganic bases such as NaH, CaH₂, LiH, LiAlH₄, NaBH₄, Cs^{t}OBu, K^{t}OBu, Na^{t}OBu, Li^{t}OBu, CsOH, KOH, NaOH, LiOH, Cs₂CO₃, K₂CO₃, Na₂CO₃, Li₂CO₃, CsHCO₃, KHCO₃, NaHCO₃, LiHCO₃, KF, CsF, tetra-n-butylammonium fluoride (TBAF), organic bases such as triethylamine, pyridine, N,N-dimethyl-4-aminopyridine (DMAP), diazabicycloundecene (DBU) and 1,4-diazabicyclo[2.2.2]octane (DABCO), and organic lithium reagents such as ⁿBuLi, ^{t}BuLi, and lithium diisopropylamide (LDA).

The reaction temperature of the above reaction is usually -80°C to 60°C, preferably -60°C to 40°C, and more preferably -50°C to 30°C.

The reaction time of the above reaction may usually be 1 hour to 5 days, for example 1-3 days.

The end functional group of the compound of formula (A) can be converted into a desired functional group by an appropriate treatment. For example, the end functional group can be converted into COOR¹¹ by reacting alcohol HOR¹¹, and the end functional group can be converted into e.g. CH₂OH or CHO by reducing the terminal functional group, whereby the compound of formula (1a) or (1b) can be obtained.

Further, the compound of formula (A) can be fluorinated. For example, the hydrogen atom bonded to the carbon of the fluoropolyether group-containing compound can be fluorinated and converted into the perfluoropolyether group-containing compound.

### Examples

The following Examples 1 and 2 describe the synthesis of compounds of the present invention. Reference Examples 3-15 describe the synthesis of compounds not claimed in the present invention.

### Example 1: (Synthesis of compound A1)

To a nitrogen-purged reaction container, 27.3 g of cesium fluoride, 662 mL of diglyme, and 198.2 g of methyl 2,2,3-trifluoro-3-oxopropanoate were added, and the mixture was stirred at 5°C for 10 minutes under an ice bath. Subsequently, at 5°C under the ice bath, 892.2 g of 2,2,3,3-tetrafluorooxetane was added dropwise from the dropping funnel to the reaction container over 20 minutes and stirred for 2 hours. Then, the ice bath was removed and the mixture was stirred for 48 hours. The obtained reaction solution was filtered under pressure with a 5 µm PTFE filter, and diglyme was evaporated to obtain compound A1.
(Number-average molecular weight: 912 (NMR), initiation efficiency from the acid fluoride: 100%)

¹⁹F-NMR assignment of compound A1

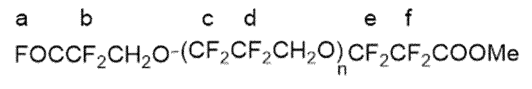

Chemical shift is based on m-xylene hexafluoride standard (-80.0 ppm)
a: 3.5 ppm, b: -130.3 ppm, c: -106.9 to -106.2 ppm, d: -140.4 to -140.7 ppm, e: -106.0 ppm, f: -137.9 ppm

### Example 2: (Synthesis of compound A2)

To a nitrogen-purged reaction container, 15.4 g of cesium fluoride, 290 mL of diglyme, and 107.0 g of methyl 2,2,3-trifluoro-3-oxopropanoate were added, and the mixture was stirred at 5°C for 10 minutes under an ice bath. Subsequently, at 5°C under the ice bath, 526.0 g of 2,2,3,3-tetrafluorooxetane was added dropwise from the dropping funnel to the reaction container over 20 minutes and stirred for 2 hours. Then, the ice bath was removed and the mixture was stirred for 50 hours. To the obtained reaction solution, 60 mL of methanol was added dropwise over 20 minutes, and the mixture was stirred for 24 hours. After evaporating volatile contents from the reaction solution under reduced pressure, 80 g of m-xylene hexafloride and 40 g of water were added for separation and washing, and 5 g of magnesium sulfate was added to the extracted organic layer for drying. Volatile contents were evaporated from the resulting treated solution to obtain compound A2.
(Number-average molecular weight: 865 (NMR), initiation efficiency from the acid fluoride: 100%)

¹⁹F-NMR assignment of compound A2

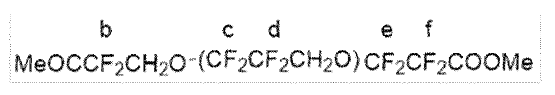

Chemical shift is based on m-xylene hexafluoride standard (-80.0 ppm)
b: -131.0 ppm, c: -106.4 to -106.8 ppm, d: -140.7 ppm, e: - 106.0 ppm, f: -138.0 ppm

### Reference Example 3: (Synthesis of compound B1)

To a nitrogen-purged reaction container, 4.23 g of cesium fluoride, 101 g of tetraglyme, and 4.22 g of methyl 2,2,3-trifluoro-3-oxopropanoate were added, and the mixture was stirred at 0°C for 2 hours. Subsequently, a total of 200 g of hexafluoropropylene oxide was added in 10 g increments over 60 minutes at 0°C, and the mixture was then stirred. The obtained reaction solution was filtered under pressure with a 5 µm PTFE filter, and tetraglyme was evaporated to obtain compound B1 (COF and methyl ester).
(Number-average molecular weight: 2,000 (NMR), initiation efficiency from the acid fluoride: 40%)

¹⁹F-NMR assignment of compound B1

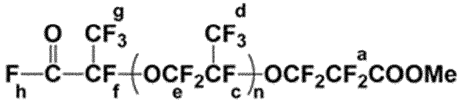

Chemical shift is based on m-xylene hexafluoride standard (-65.0 ppm)
a: -122.4 ppm, c: -145.9 to -146.3 ppm, d,e: -79.5 to -83.3 ppm, f: -131.0 ppm, g: -82.2 to -82.6 ppm, h: 25.0 ppm

### Reference Example 4: (Synthesis of compound B2)

To the obtained B1 reaction solution, 10 mL of methanol was added dropwise over 5 minutes at 0°C, and the mixture was stirred for 2 hours. For separation and washing, 130 g of m-xylene hexafloride and 200 g of water were added, and 5 g of magnesium sulfate was added to the extracted organic layer for drying. Volatile contents were evaporated from the resulting treated solution to obtain compound B2 (methyl ester at both ends).
(Number-average molecular weight: 2,000 (NMR), initiation efficiency from the acid fluoride: 40%)

¹⁹F-NMR assignment of compound B2

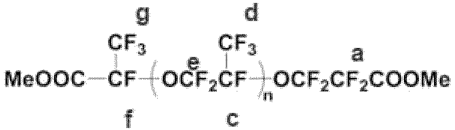

Chemical shift is based on m-xylene hexafluoride standard (-65.0 ppm)
a: -122.5 ppm, c: -145.4 to -146.2 ppm, d,e: -79.6 to -82.5 ppm, f: -132.5 ppm, g: -83.7 to -84.1 ppm

### Reference Example 5: (Synthesis of compound B3)

To a nitrogen-purged reaction container, 3.85 g of cesium fluoride, 100 g of tetraglyme, and 3.98 g of methyl 2,2,3-trifluoro-3-oxopropanoate were added, and the mixture was stirred at 0°C for 2 hours. Subsequently, a total of 200 g of hexafluoropropylene oxide was added in 5 g increments over 60 minutes at -30°C, and the mixture was then stirred. To the obtained reaction solution, 10 mL of methanol was added dropwise over 5 minutes at 0°C, and the mixture was stirred for 2 hours. For separation and washing, 130 g of m-xylene hexafloride and 200 g of water were added, and 5 g of magnesium sulfate was added to the extracted organic layer for drying. Volatile contents were evaporated from the resulting treated solution to obtain compound B3 (methyl ester at both ends).
(Number-average molecular weight: 3,000 (NMR), initiation efficiency from the acid fluoride: 70%)

### Reference Example 6: (Synthesis of compound B4)

To a nitrogen-purged reaction container, 1.94 g of potassium fluoride, 13 g of tetraglyme, 130 g of m-xylene hexafloride, and 5.20 g of methyl 2,2,3-trifluoro-3-oxopropanoate were added, and the mixture was stirred at 0°C for 2 hours. Subsequently, a total of 140 g of hexafluoropropylene oxide was added in 10 g increments over 60 minutes at 0°C, and the mixture was then stirred. The obtained reaction solution was filtered under pressure with a 5 µm PTFE filter, and tetraglyme was evaporated to obtain compound B4 (COF and methyl ester).
(Number-average molecular weight: 2,900 (NMR), initiation efficiency from the acid fluoride: 60%, Mw / Mn = 1.07 (GPC))

### Reference Example 7: (Synthesis of compound B5)

To the obtained B4 reaction solution, 10 mL of methanol was added dropwise over 5 minutes at 0°C, and the mixture was stirred for 2 hours. For separation and washing, 200 g of water was added, and 5 g of magnesium sulfate was added to the extracted organic layer for drying. Volatile contents were evaporated from the resulting treated solution to obtain compound B5 (methyl ester at both ends).
(Number-average molecular weight: 2,900 (NMR), initiation efficiency from the acid fluoride: 60%)

### Reference Example 8: (Synthesis of compound B6)

To a nitrogen-purged reaction container, 1.87 g of potassium fluoride, 13 g of tetraglyme, 130 g of m-xylene hexafloride, and 5.15 g of methyl 2,2,3-trifluoro-3-oxopropanoate were added, and the mixture was stirred at 0°C for 2 hours. Subsequently, a total of 140 g of hexafluoropropylene oxide was added in 5 g increments over 60 minutes at -30°C, and the mixture was then stirred. To the obtained reaction solution, 10 mL of methanol was added dropwise over 5 minutes at 0°C, and the mixture was stirred for 2 hours. To the reaction solution, 200 g of water was added for separation and washing, and 5 g of magnesium sulfate was added to the extracted organic layer for drying. Volatile contents were evaporated from the resulting treated solution to obtain compound B6 (methyl ester at both ends). (Number-average molecular weight: 4,000 (NMR), initiation efficiency from the acid fluoride: 86%)

### Reference Example 9: (Synthesis of compound B7)

To a nitrogen-purged reaction container, 4.23 g of cesium fluoride, 11 g of tetraglyme, 120 g of m-xylene hexafloride, and 4.22 g of methyl 2,2,3-trifluoro-3-oxopropanoate were added, and the mixture was stirred at 0°C for 2 hours. Subsequently, a total of 200 g of hexafluoropropylene oxide was added in 10 g increments over 60 minutes at 0°C, and the mixture was then stirred. To the obtained reaction solution, 10 mL of methanol was added dropwise over 5 minutes at 0°C, and the mixture was stirred for 2 hours. To the reaction solution, 200 g of water was added for separation and washing, and 5 g of magnesium sulfate was added to the extracted organic layer for drying. Volatile contents were evaporated from the resulting treated solution to obtain compound B7 (methyl ester at both ends). (Number-average molecular weight: 3,500 (NMR), initiation efficiency from the acid fluoride: 43%)

### Reference Example 10: (Synthesis of compound B8)

To a nitrogen-purged reaction container, 4.23 g of cesium fluoride, 11 g of tetraglyme, 120 g of Novec 7200 (manufactured by 3M), and 4.22 g of methyl 2,2,3-trifluoro-3-oxopropanoate were added, and the mixture was stirred at 0°C for 2 hours. Subsequently, a total of 200 g of hexafluoropropylene oxide was added in 10 g increments over 60 minutes at 0°C, and the mixture was then stirred. To the obtained reaction solution, 10 mL of methanol was added dropwise over 5 minutes at 0°C, and the mixture was stirred for 2 hours. To the reaction solution, 200 g of water was added for separation and washing, and 5 g of magnesium sulfate was added to the extracted organic layer for drying. Volatile contents were evaporated from the resulting treated solution to obtain compound B8 (methyl ester at both ends). (Number-average molecular weight: 3,400 (NMR), initiation efficiency from the acid fluoride: 45%)

### Reference Example 11: (Synthesis of compound B9)

To a nitrogen-purged reaction container, 4.23 g of cesium fluoride, 11 g of tetraglyme, 135 g of Novec 7100 (manufactured by 3M), and 4.22 g of methyl 2,2,3-trifluoro-3-oxopropanoate were added, and the mixture was stirred at 0°C for 2 hours. Subsequently, a total of 200 g of hexafluoropropylene oxide was added in 10 g increments over 60 minutes at 0°C, and the mixture was then stirred. To the obtained reaction solution, 10 mL of methanol was added dropwise over 5 minutes at 0°C, and the mixture was stirred for 2 hours. To the reaction solution, 200 g of water was added for separation and washing, and 5 g of magnesium sulfate was added to the extracted organic layer for drying. Volatile contents were evaporated from the resulting treated solution to obtain compound B9 (methyl ester at both ends). (Number-average molecular weight: 3,500 (NMR), initiation efficiency from the acid fluoride: 45%)

### Reference Example 12: (Synthesis of compound B10)

To a nitrogen-purged reaction container, 4.23 g of cesium fluoride, 11 g of tetraglyme, 115 g of 1,1,1,3,3-pentafluorobutane, and 4.22 g of methyl 2,2,3-trifluoro-3-oxopropanoate were added, and the mixture was stirred at 0°C for 2 hours. Subsequently, a total of 200 g of hexafluoropropylene oxide was added in 10 g increments over 60 minutes at 0°C, and the mixture was then stirred. To the obtained reaction solution, 10 mL of methanol was added dropwise over 5 minutes at 0°C, and the mixture was stirred for 2 hours. To the reaction solution, 200 g of water was added for separation and washing, and 5 g of magnesium sulfate was added to the extracted organic layer for drying. Volatile contents were evaporated from the resulting treated solution to obtain compound B10 (methyl ester at both ends). (Number-average molecular weight: 2,500 (NMR), initiation efficiency from the acid fluoride: 40%)

### Reference Example 13: (Synthesis of compound B11)

To a nitrogen-purged reaction container, 2.9 g of potassium fluoride, 101 g of tetraglyme, and 9 g of methyl 2,2,3-trifluoro-3-oxopropanoate were added, and the mixture was stirred at 0°C for 2 hours. Subsequently, a total of 200 g of hexafluoropropylene oxide was added in 10 g increments over 60 minutes at 0°C, and the mixture was then stirred. To the obtained reaction solution, 10 mL of methanol was added dropwise over 5 minutes at 0°C, and the mixture was stirred for 2 hours. To the reaction solution, 200 g of water was added for separation and washing, and 5 g of magnesium sulfate was added to the extracted organic layer for drying. Volatile contents were evaporated from the resulting treated solution to obtain compound B11 (methyl ester at both ends). (Number-average molecular weight: 900 (NMR), initiation efficiency from the acid fluoride: 74%)

### Reference Example 14: (Synthesis of compound B12)

To a nitrogen-purged reaction container, 2.9 g of potassium fluoride, 20 g of tetraglyme, 120 g of m-xylene hexafloride, and 9 g of methyl 2,2,3-trifluoro-3-oxopropanoate were added, and the mixture was stirred at 0°C for 2 hours. Subsequently, a total of 200 g of hexafluoropropylene oxide was added in 10 g increments over 60 minutes at 0°C, and the mixture was then stirred. To the obtained reaction solution, 10 mL of methanol was added dropwise over 5 minutes at 0°C, and the mixture was stirred for 2 hours. To the reaction solution, 200 g of water was added for separation and washing, and 5 g of magnesium sulfate was added to the extracted organic layer for drying. Volatile contents were evaporated from the resulting treated solution to obtain compound B12 (methyl ester at both ends). (Number-average molecular weight: 2,100 (NMR), initiation efficiency from the acid fluoride: 76%)

### Reference Example 15: (Synthesis of compound B13)

To a nitrogen-purged reaction container, 2.9 g of potassium fluoride, 20 g of tetraglyme, 120 g of Novec 7200 (manufactured by 3M), and 9 g of methyl 2,2,3-trifluoro-3-oxopropanoate were added, and the mixture was stirred at 0°C for 2 hours. Subsequently, a total of 200 g of hexafluoropropylene oxide was added in 10 g increments over 60 minutes at 0°C, and the mixture was then stirred. To the obtained reaction solution, 10 mL of methanol was added dropwise over 5 minutes at 0°C, and the mixture was stirred for 2 hours. To the reaction solution, 200 g of water was added for separation and washing, and 5 g of magnesium sulfate was added to the extracted organic layer for drying. Volatile contents were evaporated from the resulting treated solution to obtain compound B13 (methyl ester at both ends). (Number-average molecular weight: 1,900 (NMR), initiation efficiency from the acid fluoride: 75%)

### Industrial Applicability

According to the present method, a fluoropolyether group-containing compound having functional groups at both ends can be easily produced.

## Claims

1. A compound, which is a fluoropolyether-containing compound of formula (1a) or (1b):
R^{a}-R²-R¹-R^{b} (1a)
R^{a}-R²-R¹-R^{b} (1b)
wherein
R^{a}, R^{b} each independently are COF, COOR¹¹, CH₂OH or CHO, wherein R¹¹ each independently is H or C₁₋₆-alkyl optionally substituted with F;
R¹ is C₂₋₁₀-alkylene optionally substituted with F;
and
R² is -R^{6a}- (OR^{5a})ₙ-O-, wherein R^{5a} is linear C₂₋₁₀-fluoroalkylene, R^{6a} is linear C₁₋₉-fluoroalkylene, and n is an integer of 2-200,
provided that in formula (1a) R^{a} and R^{b} are not the same, and in formula (1b) R^{5a} is CF₂CF₂CH₂ and R^{6a} is CF₂CH₂.

2. The compound of claim 1, wherein in formula (1a) R^{5a} is C₂₋₄-fluoroalkylene and R^{6a} is C₁₋₃-fluoroalkylene.

3. The compound of claim 1 or 2,wherein in formula (1a) R^{5a} is C₃-fluoroalkylene and R^{6a} is C₂-fluoroalkylene.

4. The compound of any of claims 1-3, wherein in formula (1a) R^{5a} is CF₂CF₂CH₂ or CF₂CF₂CF₂.

5. The compound of any of claims 1-5, wherein R^{a} is COF, R^{b} is COOR¹¹, and R¹¹ is C₁₋₆-alkyl.

6. The compound of any of claims 1-6, wherein R¹ is C₂₋₁₀-perfluoroalkylene.

7. A method for producing a compound of formula (A):
FOC-R²R¹-COOR¹² (A)
wherein
R¹ is C₂₋₁₀-alkylene optionally substituted with F;
R² is -R⁶-(OR⁵)ₙ-O-, wherein R⁵ is linear C₂₋₁₀-fluoroalkylene, R⁶ is linear C₁₋₉-fluoroalkylene, and n is an integer of 2-200; and
R¹² is C₁₋₆-alkyl optionally substituted with F;
comprising reacting an acid fluoride compound of the formula (a):
FOC-R¹³-COOR¹² (a)
wherein
R¹³ is C₁₋₉-alkylene optionally substituted with F; and
R¹² is C₁₋₆-alkyl optionally substituted with F,
with a cyclic ether compound of formula (b): 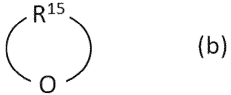 wherein R¹⁵ is linear C₂₋₁₀-fluoroalkylene.

8. The method of claim 7, wherein R¹³ is
C₁₋₉-perfluoroalkylene.

9. The method of claim 7 or 8, wherein R¹⁵ is linear C₂₋₄-fluoroalkylene.

10. The method of any of claims 7-9, wherein R¹⁵ is CF₂CF₂CH₂.

## Patentansprüche

1. Verbindung, die eine Fluorpolyether-haltige Verbindung der Formel (1a) oder (1b) ist:
R^{a}-R²-R¹-R^{b} (1a)
R³-R²-R¹-R^{b} (1b)
worin
R^{a}, R^{b} jeweils unabhängig voneinander COF, COOR¹¹, CH₂OH oder CHO sind, worin R¹¹ jeweils unabhängig voneinander H oder C₁₋₆-Alkyl ist, das optional mit F substituiert ist;
R¹ C₂₋₁₀-Alkylen ist, das optional mit F substituiert ist; und
R² -R^{6a}-(OR^{5a})ₙ-O- ist, worin R^{5a} lineares C₂₋₁₀-Fluoralkylen ist, R^{6a} lineares C₁₋₉-Fluoralkylen ist und n eine ganze Zahl von 2 bis 200 ist,
vorausgesetzt, dass in Formel (1a) R^{a} und R^{b} nicht gleich sind und in Formel (1b) R^{5a} CF₂CF₂CH₂ ist und R^{6a} CF₂CH₂ ist.

2. Verbindung gemäß Anspruch 1, worin in Formel (1a) R^{5a} C₂₋₄-Fluoralkylen ist und R^{6a} C₁₋₃-Fluoralkylen ist.

3. Verbindung gemäß Anspruch 1 oder 2, worin in Formel (1a) R^{5a} C₃-Fluoralkylen ist und R^{6a} C₂-Fluoralkylen ist.

4. Verbindung gemäß einem der Ansprüche 1-3, worin in Formel (1a) R^{5a} CF₂CF₂CH₂ oder CF₂CF₂CF₂ ist.

5. Verbindung gemäß einem der Ansprüche 1-5, worin R^{a} COF ist, R^{b} COOR¹¹ ist und R¹¹ C₁₋₆-Alkyl ist.

6. Verbindung gemäß einem der Ansprüche 1-6, worin R¹ C₂₋₁₀-Perfluoralkylen ist.

7. Verfahren zur Herstellung einer Verbindung der Formel (A) :
FOC-R²R¹-COOR¹² (A)
worin
R¹ C₂₋₁₀-Alkylen ist, das optional mit F substituiert ist;
R2 -R⁶-(OR⁵)ₙ-O- ist, worin R⁵ lineares C₂₋₁₀-Fluoralkylen ist, R⁶ lineares C₁₋₉-Fluoralkylen ist und n eine ganze Zahl von 2 bis 200 ist; und
R12 C₁₋₆-Alkyl ist, das optional mit F substituiert ist;
umfassend das Umsetzen einer Säurefluoridverbindung der Formel (a):
FOC-R¹³-COOR¹² (a)
worin
R¹³ C₁₋₉-Alkylen ist, das optional mit F substituiert ist; und
R¹² C₁₋₆-Alkyl ist, das optional mit F substituiert ist,
mit einer cyclischen Etherverbindung der Formel (b): 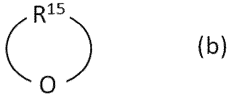 worin R¹⁵ lineares C₂₋₁₀-Fluoralkylen ist.

8. Verfahren gemäß Anspruch 7, worin R¹³ C₁₋₉-Perfluoralkylen ist.

9. Verfahren gemäß Anspruch 7 oder 8, worin R¹⁵ lineares C₂₋₄-Fluoralkylen ist.

10. Verfahren gemäß einem der Ansprüche 7-9, worin R¹⁵ CF₂CF₂CH₂ ist.

## Revendications

1. Composé, qui est un composé contenant un groupe fluoropolyéther de formule (1a) ou (1b) :
R^{a}-R²-R¹-R^{b} (1a)
R^{a-}R²-R¹-R^{b} (1b)
dans laquelle
R^{a}, R^{b} sont chacun indépendamment COF, COOR¹¹, CH₂OH ou CHO, dans laquelle R¹¹ est chacun indépendamment H ou un alkyle en C₁₋₆ facultativement substitué par F ;
R¹ est un alkylène en C₂₋₁₀ facultativement substitué par F ; et
R² est -R^{6a}-(OR^{5a})ₙ-O-, dans laquelle R^{5a} est un fluoroalkylène en C₂₋₁₀ linéaire, R^{6a} est un fluoroalkylène en C₁₋₉ linéaire, et n est un nombre entier de 2-200,
à condition que dans la formule (1a) R^{a} et R^{b} ne soient pas les mêmes, et dans la formule (1b) R^{5a} soit CF₂CF₂CH₂ et R^{6a} soit CF₂CH₂.

2. Composé selon la revendication 1, dans lequel, dans la formule (1a), R^{5a} est un fluoroalkylène en C₂₋₄ et R^{6a} est un fluoroalkylène en C₁₋₃.

3. Composé selon la revendication 1 ou la revendication 2, dans lequel, dans la formule (1a), R^{5a} est un fluoroalkylène en C₃ et R^{6a} est un fluoroalkylène en C₂.

4. Composé selon l'une quelconque des revendications 1 à 3, dans lequel, dans la formule (1a), R^{5a} est CF₂CF₂CH₂ ou CF₂CF₂CF₂.

5. Composé selon l'une quelconque des revendications 1 à 5, dans lequel R^{a} est COF, R^{b} est COOR¹¹et R¹¹ est un alkyle en C₁₋₆.

6. Composé selon l'une quelconque des revendications 1 à 6, dans lequel R¹ est un perfluoroalkylène en C₂₋₁₀.

7. Procédé de production d'un composé de Formule (A) :
FOC-R²-R¹-COOR¹² (A)
dans laquelle
R¹ est un alkylène en C₂₋₁₀ facultativement substitué par F ;
R² est -R⁶-(OR⁵)ₙ-O-, dans laquelle R⁵ est un fluoroalkylène en C₂₋₁₀ linéaire, R⁶ est un fluoroalkylène en C₁₋₉ linéaire, et n est un nombre entier de 2-200 ; et
R¹² est un alkyle en C₁₋₆ facultativement substitué par F ;
comprenant la réaction d'un composé de fluorure d'acide de formule (a) :
FOC-R¹³-COOR¹² (a)
dans laquelle
R¹³ est un alkylène en C₁₋₉ facultativement substitué par F ; et
R¹² est un alkyle en C₁₋₆ facultativement substitué par F,
avec un composé d'éther cyclique de formule (b) : 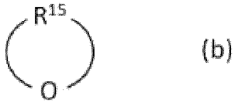 dans laquelle R¹⁵ est un fluoroalkylène en C₂₋₁₀ linéaire.

8. Procédé selon la revendication 7, dans lequel R¹³ est un perfluoroalkylène en C₁₋₉.

9. Procédé selon la revendication 7 ou la revendication 8, dans lequel R¹⁵ est un fluoroalkylène en C₂₋₄ linéaire.

10. Procédé selon l'une quelconque des revendications 7 à 9, dans lequel R¹⁵ est CF₂CF₂CH₂.
